# EUROPEAN PATENT SPECIFICATION

(11) **EP 2 021 033 B1**
(45) Date of publication and mention of the grant of the patent: **28.03.2012**
(21) Application number: 07734572.6
(22) Date of filing: 15.05.2007
(51) Int. Cl.: C07K 16/24, A61K 38/20, A61K 39/00

(54) **IL-1-ALPHA FOR USE IN A METHOD OF TREATMENT OF ATHEROSCLEROTIC PLAQUES.**
IL-1-ALPHA FÜR DEN EINSATZ IN EINEM VERFAHREN ZUR BEHANDLUNG VON ATHEROSKLEROTISCHEN PLAQUES.
IL-1-ALPHA POUR UNE UTILISATION DANS UNE MÉTHODE DE TRAITEMENT DES PLAQUES D'ATHÉROSCLÉROSE.

(30) Priority: 15.05.2006 US 800029 P
(43) Date of publication of application: 11.02.2009
(73) Proprietor: XBiotech, Inc, Vancouver, British Columbia V6E 2E9 (CA)
(72) Inventor: SIMARD, John, Austin, TX 78733 (US)
(74) Representative: Tombling, Adrian George
(86) International application number: PCT/IB2007/001264
(87) International publication number: WO 2007/132338

(56) References cited:
- WO-A1-2007/015128
- WO-A1-2007/039552
- WO-A2-2004/100987
- US-B2- 6 555 579
- MARIOTTI MASSIMO ET AL: "Interleukin 1 alpha is a marker of endothelial cellular senescent" IMMUNITY AND AGEING, BIOMED CENTRAL, LONDON, GB, vol. 3, no. 1, 6 April 2006 (2006-04-06), page 4, XP021019190 ISSN: 1742-4933
- MERHI-SOUSSI F. ET AL.: 'Interleukin-1 Plays A Major Role In Vascular Inflammation And Atherosclerosis In Male Apolipoprotein E-Knockout Mice' CARDIOVASC. RES. vol. 66, 01 June 2005, pages 583 - 593, XP004898932
- SVENSON M. ET AL.: 'Cytokine Vaccination: Neutralising IL-1alpha Autoantibodies Induced By Immunisation With Homologous IL-1alpha' J. IMMUNOL. METHODS vol. 236, 06 March 2000, pages 1 - 8, XP004190741

## Description

### BACKGROUND OF THE INVENTION

IL-1α is well characterized as a primary mediator of inflammation and its role in inflammatory related disease has been suggested in several animal models. Human IgG autoantibodies (aAb) against interleukin (IL)-1α have been detected with a relatively high frequency in the general population. In fact, it has been reported that more than 20% of ostensibly healthy persons have highly specific IL-1α aAb. Although observations of men with natural IL-1α aAb have suggested a role for neutralization of endogenous IL-1α in reduced risk of progression of inflammatory related diseases, such as atherosclerosis or rheumatoid arthritis, these studies had not rules out the presence of other autoantibodies in these individuals, and it has been difficult to establish a causal link and a physiological role of these anti-IL-1α antibodies has not been clearly established.

WO2004/100987 discloses the use of an IL-1 antagonist in the treatment of neointimal hyperplasia.

Merhi-Soussi et al., (Cardiovasc. Res., 66, 583-593, 2005) discloses that IL-1 plays a role in atherosclerosis in male apolipoprotein E knockout mice.

Svenson et al., (J. Immunol. Methods, 236, 1-8, 2000) discloses that IL-1α can be used to immunise and generate IL-1α neutralising autoantibodies.

Massimo et al., (Immunity and Ageing, 3, 4, 2006) discloses that IL-1α is a marker for endothelial cellular senescent.

W02007/015128 and WO2007/039552 are citable under Article 54(3)EPC only and relate to the use of IL-1α autoantibodies for treating atherosclerosis and the use of IL-1 for treating a variety of diseases, respectively.

### BRIEF DESCRIPTION OF THE DRAWINGS

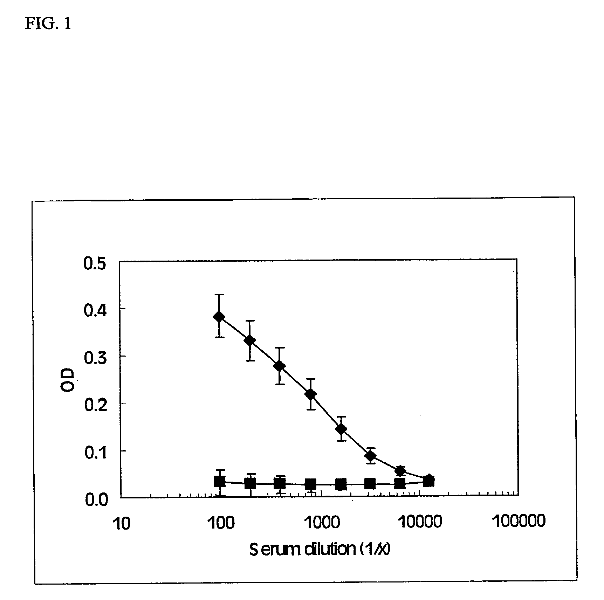

FIG. 1. Anti-IL-1α autoantibody formation on day 56 in C57BL/6 mice after three subcutaneous injections with IL-1α-PPD conjugate in alum (◆). Control mice immunized with PPD in alum only (■).

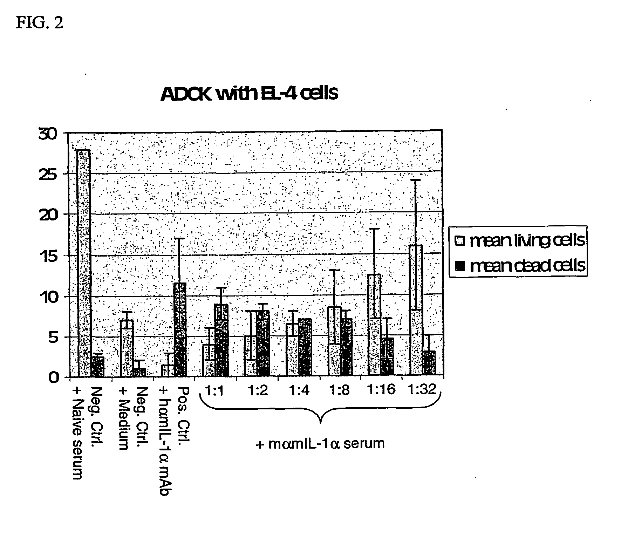

FIG. 2. Antibody-dependent complement-mediated killing of EL-4 cells. EL-4 cells were incubated with serial dilutions of mouse anti-mouse IL-α polyclonal antiserum. The ratio of killed cells to viable cells is proportional to the serum concentration. A human anti-mouse IL-1α monoclonal antibody was used as a positive control. Incubation with naive murine serum or with culture medium alone served as the two negative controls.

### DETAILED DESCRIPTION OF THE INVENTION

The ApoE-/- mice have an engineered lipid transport defect that results in rapid progression of atherosclerosis-like plaques in major arteries. These mice are considered the most compelling model for human atherosclerosis, because they are hypercholesterolemic and spontaneously develop arterial lesions. The ApoE-/- mice have consequently been extensively used as a model system for studying atherosclerosis and treatments.

An animal model for antibody neutralization of IL-1α can be obtained, e.g., by immunizing ApoE-/- mice against IL-1α. All immunized animals develop IgG aAb to IL-1α, which persists at high levels. The IL-1α aAb from sera of immunized mice inhibits binding of IL-1α to NOB-1, an IL-1α responsive murine T cell line, and neutralizes IL-1α (but not IL-1β-induced IL-6) *in vivo.*

Control ApoE-/- mice which are fed a high fat diet develop atherosclerosis-like lesions in major arteries. The lesions are marked by macrophage infiltration, a necrotic core and proliferating smooth muscle cells with varying amounts of extracellular matrix. In contrast, ApoE-/- animals immunized against IL-1α have drastically reduced levels of atherosclerotic lesions and a striking resistance to progression of atherosclerosis. In mice which have fatty streaks (the beginning of atherosclerotic lesions) before immunization, immunization with IL-1α arrests the development of atherosclerotic lesions, such that the vascular bed remains essentially healthy.

ApoE-/- mice are well protected against atherosclerosis-related disorders (e.g., peripheral ischemic heart disease, coronary artery disease, cerebrovascular disease, peripheral arterial disease) by the presence of endogenous IL-1α autoantibody generated through immunization. The animal model supports our earlier clinical observations that men with natural IL-1α aAb have a reduced incidence of atherosclerosis-related heart disease compared to men who do not have neutralizing IL-1α aAb.

Because humans who naturally produce IL-1α aAb have been found to be less risk for the development of atherosclerosis, it seems likely that natural IL-1α aAb may play a physiological role in neutralizing the delaterious inflammatory effects of IL-1α in the vascular endothelium. Thus, the invention also provides a method of treating atherosclerotic plaques in a mammal by inducing protective IL-1α auto-antibodies against the disease. Clinical observations of IL-1α autoantibodies in about 20% of the population, with no apparent health defects, suggests that administration of neutralizing autoantibodies against IL-1α would not pose a health risk. Moreover, IL-1α knockout mice also are apparently healthy, supporting this approach as safe. Induction of IL-1α aAb in humans is therefore a safe and effective way to reduce the risk and severity of atherosclerosis-related diseases.

Any methods of immunization known in the art can be used to achieve the desired autoantibody response in either animal models or mammalian (e.g., cats, dogs, sheep, pigs, goats; preferably human) patients (see below).

| ADJUVANT | EXAMPLE |
|---|---|
| Inorganic Salt | Aluminium hydroxide, calcium phosphate, beryllium hydroxide |
| Delivery systems | Incomplete Freund's adjuvant |
| Bacterial Products | Complete Freund's Adjuvant, BCG, plasmid |
| DNA | CpG motifs |
| Immune Stimulatory Complexes (ISCOMS) | Mixture of Quil A containing viral proteins |
| Cytokines | GM-CSF, IL-12, IL-1, IL-2 |
| Recombinant Virus | Influenza |
| Virus-like particle conjugate | 2/6 VLP containing bovine rotavirus VP2 and human rotavirus VP6 |
| Recombinant Bacteria | Attenuated Salmonella typhimurium |

The above disclosure generally describes the present invention. A more complete understanding can be obtained by reference to the following specific examples, which are provided for purposes of illustration only and are not intended to limit the scope of the invention.

### EXAMPLE 1

### ApoE Knockout Mice

The ApoE-/- mice are obtained from Jackson Laboratory, Bar Harbor, ME. Only male animals are used to avoid possible influence of gender on the development of vascular lesions; moreover, clinical studies observing a protective role for IL-1α Aab in progression of atherosclerosis have been made, to this point, only in men. Ten week-old mice are used and fed a diet with high cholesterol content (1.25% cholesterol, 0% cholate; Research Diets, New Brunswick, NJ). The mice are fed the diet for 10 weeks and then sacrificed. Blood is sampled and aortas are perfused, cut into parts, and either fixed or frozen according to standard methods.

### EXAMPLE 2

### Immunization with Murine IL-1α

Mice are immunized with murine IL-1α conjugated to purified protein derivative of tuberculin (PPD) at a ratio of 0.41 (w/w) according to the method described by Svenson et al., J Immunol Methods. 2000 Mar 6;2361(1-2):1-8. Mice are inoculated with subcutaneous injections in the base of the tail. Inoculations are repeated three times, three weeks apart. To analyze IL-1α aAb, mice are bled from the retroorbital plexus 2 weeks after each injection. Control animals receive identical inoculation schedule with a PPD solution containing no IL-1α.

### EXAMPLE 3

### Assays

Mouse IgG responses to IL-1α are determined as described by Svenson *et al.,* 2000. Saturation binding analysis of IL-1α to IgG is performed as described (Svenson et al., J Clin Invest. 1993 Nov;92(5):2533-9). Identical samples are run in parallel on the protein G Sepharose columns and columns containing Sephadex G-75 superfine (Svenson et al., Cytokine. 1992 Mar;4(2):125-33) to compare the ¹²⁵I-IL-1α bound to serum IgG with the total binding to serum.

Cellular receptor assays are performed using the NOB-1 murine T cell line as described in Svenson *et al.,* 2000. IL-1α RIAs and IL-6 ELISAs also are performed as described in Svenson *et al.,* 2000.

*In* vivo induction of IL-6 is performed as described in *Svenson et al.,* 2000.

### EXAMPLE 4

### Absence of natural anti IL-1α aAb in ApoE-/- mice

Sera from 15 ApoE-/- mice aged 10 weeks to 10 months are all negative for IgG anti-IL-1α aAb.

### EXAMPLE 5

### Generation of IL-1α aAb in ApoE-/- mice

After four inoculations with IL-1α conjugated to PPD, all mice have high IL-1α IgG aAb titers. No aAb are found in sera of control mice inoculated with PPD alone. There is no significant weight difference between the groups at 3 months after vaccination.

### EXAMPLE 6

### Characterization of induced IL-1α aAb

Sera are collected 2 weeks and 6 weeks after vaccination of the positive mice are tested. No difference is seen between total IL-1α binding to serum and binding to IgG. The K_{d}s range from 0.1 nM to 1.3 nM (*e.g*., 0.1, 0.15, 0.2, 0.25, 0.3, 0.4, 0.5, 0.6, 0.7, 0.8, 0.9, 1.0, 1.1, 1.2, 1.3 nM).

### EXAMPLE 7

### Characteristics of induced anti IL-1α aAb

IL-1α aAb are tested using an RIA. The antisera function similarly to those disclosed in Svenson *et al.,* 2000.

### EXAMPLE 8

### Suppression of receptor binding

The binding of ¹²⁵I-IL-1α to the murine cell-line NOB-1 is suppressed by at least 10% (*e.g*., at least 10, 15, 20, 25, 30, 35, 40, 45, 50, 55, 60, 65, 70, 75, 80, 85, 90, or 95%) by all aAb-positive sera collected two weeks after vaccination and tested as described in *Svenson et al.,* 2000. aAb-negative controls are negative.

### EXAMPLE 9

### In vivo neutralization of IL-1α

Neutralizing activity of representative antisera are tested as described in Svenson *et al.,* 2000. Data indicate that IL-1α aAb neutralize IL-1α activity in NOB-1 cells.

### EXAMPLE 10

### Analysis of atherosclerotic lesions

Mice are sacrificed at different time points, and the extent of atherosclerosis is evaluated. Plaque deposition and atherosclerotic lesions are assessed in aortic roots and thoracoabdominal aortas and quantified according to standardized methods (*e.g*., Trogan et al., Proc Natl Acad Sci U S A. 2002 Feb 19;99(4):2234-9; Chaabane et al., Invest Radiol. 2003 Aug;38(8):532-8). Aortic root atherosclerotic lesion areas in IL-1α-immunized ApoE -/- mice are significantly decreased as compared to ApoE -/- control mice (*e.g*., by at least 10, 15, 20, 25, 30, 35, 40, 45, 50, 55, 60, 65, 70, 75, 80, 85, 90, or 95%). Atherosclerotic lesion development is also examined in preparations of the descending aorta stained with Sudan IV. The formation of sudanophilic lipid-rich lesions in abdominal aortas of IL-1α-immunized ApoE -/- mice decreases significantly compared to their littermate control groups (*e.g*., by at least 10, 15, 20, 25, 30, 35, 40, 45, 50, 55, 60, 65, 70, 75, 80, 85, 90, or 95%). Similarly, the formation of atherosclerotic lesions in aortic arch sections, which appears after staining with hematoxylin-eosin, are significantly reduced in IL-1α immunized animals compared to controls (*e.g*., by at least 10, 15, 20, 25, 30, 35, 40, 45, 50, 55, 60, 65, 70, 75, 80, 85, 90, or 95%).

Luminal area of coronary arteries are significantly diminished in control ApoE-/- mice (*e.g*., by at least 10, 15, 20, 25, 30, 35, 40, 45, 50, 55, 60, 65, 70, 75, 80, 85, 90, or 95%) compared to control mice. Histological analysis of aortic roots demonstrates the presence of CD68-positive cells in the neointima in ApoE -/- controls but not in IL-1α immunized animals.

### EXAMPLE 11

### Materials and Methods

### Measurement of anti IL-1α antibody titers by ELISA

Human or murine IL-1α, respectively, are incubated on 96 well ELISA plates over night, using 0.5µg/ml with a volume of 100µl per well. The plates are then washed 4 times with phosphate buffered saline (PBS) + 0,05% Tween 20, then saturated with a blocking solution containing 1% bovine serum albumin (BSA) in PBS + 0.05% Tween 20. Two hundred µl of this blocking buffer are used per well for 1-2 hours at room temperature. Then plates are washed again 4 times with PBS + 0.05% Tween 20 (PBST). One-hundred ml of serially diluted serum samples (1:2 dilutions in PBST + 1% BSA) are then added and incubated for one hour at room temperature or at 4°C over night. Then plates are washed again 4 times with PBST. Horseradish peroxidise (HRP) coupled anti-F_{C} antibody is then added as a secondary antibody (dilute 1:2000 in PBST with 1% BSA in, 100 µl per well, 1 hour, room temperature). Human: 0.2 µl goat anti human IgG-HRP in 400µl PBST + 1%BSA. Mouse: 0.5µl HRP goat anti mouse IgG (H+L). Then plates are washed again 4 times with PBST. The coloring reaction is made with ABTS buffer. ABTS buffer (3-ethylbenzthiazoline-6-sulfonic acid, Sigma Cat. No. A-1888, 150 mg, 0.1 M citric acid, Fisher anhydrous, Cat. No. A-940, in 500 ml; the pH is adjusted to 4.35 with NaOH pellets, and 11 ml aliquots are stored at -20°C, 40% SDS (80 g SDS in 200 ml dd H₂O), with the addition of 200 ml DMF (N,N-dimethyl formamide)). One hundred µl of the ABTS buffer are added to each well. The reaction is stopped by adding 100 µl of 2% oxalic acid solution when good contrast is visible. The optical density is then measured with an ELISA reader at a wavelength of 405nm.

### Mice

ApoE-/- mice were obtained from Jackson Laboratory (Bar Harbor, Maine, strain B6.129P2-Apoe^{tm1Unc}/J). Mice homozygous for the Apoe^{tm1Unc} mutation show a marked increase in total plasma cholesterol levels that are unaffected by age or sex. Fatty streaks in the proximal aorta are found at 3 months of age. The lesions increase with age and progress to lesions with less lipid but more elongated cells, typical of a more advanced stage of pre-atherosclerotic lesion. Moderately increased triglyceride levels have been reported in mice with this mutation on a mixed C57BL/6 x 129 genetic background. Aged ApoE deficient mice (>17 months) have been shown to develop xanthomatous lesions in the brain consisting mostly of crystalline cholesterol clefts, lipid globules, and foam cells. Smaller xanthomas were seen in the choroid plexus and ventral fornix. Recent studies indicate that ApoE deficient mice have altered responses to stress, impaired spatial learning and memory, altered long term potentiation, and synaptic damage. C57BL/6 and SCID mice were obtained from Harlan (Horst, the Netherlands).

### Immunization of mice with IL-1α and IL-1β conjugated with PPD

IL-1α and IL-1β were obtained from eBioscience (San Diego, CA). PPD was obtained from the Statens Serum Institute (Copenhagen, Denmark). The method for conjugation was adapted from *Svenson et al.* (Svenson M. 2000). IL-1α or IL-1b were incubated for 48 h at 4°C with PPD at a ratio of 0.41 (w/w) and in the presence of 0.1 % glutaraldehyde (IL-1/PPD = 0.41). As a control, PPD was treated in parallel but without IL-1α or IL-1β. The conjugate was then adsorbed to Al(OH)₃ (Rehydragel; Reheis Chemical, Dublin, Ireland) so that there was 1.5% Al(OH)₃ in the final volume.

Incubation with Alum was for 90 minutes at room temperature. The particles were then washed with 0.9% NaCl and resuspended it in 0.9% NaCl at 11 µg IL-1α/100 µl suspension, assuming a 70% adsorption of IL-1α to Al(OH)₃ (found in pilot studies using ¹²⁵I-IL-1α). The IL-1β conjugate was prepared the same way. Control suspensions were diluted identically to match the amount of PPD in the IL-1α-PPD conjugate. The conjugates were stored at 4°C until use.

### EXAMPLE 12

### Generation of an anti-IL-1α antibody response in C57BL/6 mice

As the immune system is tolerant against self-proteins such as cytokines, active vaccination has to break self tolerance. In case of most self proteins, immune tolerance is caused by a lack of specific T cells as a consequence of negative selection in the thymus. In contrast, potentially self-reactive B cells are usually present. When injecting the self-protein like IL-1α alone, these B cells do not respond, due to the lack of T cell help. Coupling a foreign protein such as PPD to the self antigen IL-1α, T cell help for the B cell stimulation is provided, because the T cells recognize PPD which results in antibody production of stimulated B cells against IL-1α and PPD.

Therefore, we vaccinated mice with an IL-1α-PPD conjugate in alum to ensure effective T-cell help for the IL-1α-specific B-cells. Antibody titers were determined by ELISA. Groups of 5 mice received subcutaneous immunizations with 15 µg of recombinant IL-1α conjugated to 10 µg-PPD using an incubation step with glutaraldehyde. The IL-1α-PPD conjugate is then absorbed to alum. Mice received three such subcutaneous immunizations with 2 weeks time interval. This immunization generated high titers of anti-IL-1α antibodies, whereas the control mice immunized with PPD in alum failed to induce detectable antibody titers (FIG. 1). Induction of anti-IL-1α antibodies required at least 2 injections. After only one injection of recombinant IL-1α-PPD conjugate in alum no antibody response was detected in sera. But after a third injection of recombinant IL-1α-PPD conjugate in alum all vaccinated mice produced anti-IL-1α antibodies.

### EXAMPLE 13

### Active immunization against IL-1α prevents formation of atherosclerosis

ApoE knock out mice (age 6 weeks) were actively immunized with 15 µg murine IL-1α conjugated with 10 µg PPD (purified protein derivate from M. tuberculosis) in aluminium hydroxide on days 0, 14 and 28 by subcutaneous administration in the neck region. The injection volume was 100 µl, and the amount of aluminium hydroxide was approximately 1 mg. Control mice were treated similarly but with a preparation that contained the same amount of PPD and aluminium hydroxide but that did not contain IL-1α. Blood was sampled from the tail vain on days 0, 28, 42, and 56 for measuring the anti-IL1α antibody response by ELISA. Four weeks after the first immunization, mice were started on an atherogenic diet with food pellets containing 16% fat, 1.16% cholesterol and 0.5 % cholic acid, a diet known to accelerate atherosclerosis. Mice were then euthanized at 18 weeks of age. Their aorta was removed for macroscopic and microscopic analysis. Histology slides were stained using Haematoxylin and Eosin (HE), as well as Sudan.

After this time point, inspection of the cut open aorta under a binocular microscope showed a marked reduction of atherosclerotic plaques in ApoE-/- mice actively immunized against IL-1α, but not in ApoE-/- control mice immunized against PPD only.

### EXAMPLE 14

### Passive immunization against IL-1α prevents formation of atherosclerosis

C57BL/6 mice were actively immunized against IL-1α with 3 subcutaneous injections of IL-1α-PPD conjugate in alum. After 56 days their serum was collected and generation of anti-IL-1α autoantibody titers were confirmed by ELISA. 200 µl of such serum was passively transferred to 6 weeks old ApoE knock out mice. These passive serum transfers were repeated every week. Control ApoE-/- mice received passive weekly passive transfers of serum from naïve C57BL/6 mice. Starting with these passive serum transfers, the ApoE-/- mice were fed an atherogenic diet with food pellets containing 16% fat, 1.16% cholesterol and 0.5 % cholic acid, in order to accelerate the formation of atherosclerosis. Control ApoE-/- mice were passively transferred 200 ml of serum from naive C57BL/6 mice in weekly intervals. Mice were euthanized on after 6 weeks for macroscopic and histological analysis of the aorta. Histological analysis included haematoxylin and eosin staining of cross sections, as well as Sudan stains.

After these 6 weeks, inspection of the cut open aorta under a binocular microscope showed a marked reduction of atherosclerotic plaques in ApoE-/- mice passively transferred anti-IL-1α antiserum, but not in ApoE-/- control mice receiving naïve serum.

### EXAMPLE 15

### Active immunization against IL-1β remained without effect on atherosclerosis

ApoE knock out mice (age 6 weeks) were actively immunized with 15 µg murine IL-1β conjugated with 10 µg PPD (purified protein derivate from *M*. *tuberculosis)* in aluminium hydroxide on days 0, 14 and 28 by subcutaneous administration in the neck region. The injection volume was 100 µl, and the amount of aluminium hydroxide was approximately 1 mg. Control mice were treated similarly but with a preparation that contained the same amount of PPD and aluminium hydroxide but that did not contain IL-1β. Blood was sampled from the tail vain on days 0, 28, 42, and 56 for measuring the anti-IL-1β antibody response by ELISA. Four weeks after the first immunization, mice were started on an atherogenic diet with food pellets containing 16% fat, 1.16% cholesterol and 0.5 % cholic acid, a diet known to accelerate atherosclerosis. Mice were then euthanized at 18 weeks of age. Their aorta was removed for macroscopic and microscopic analysis. Histology slides were stained using Haematoxylin and Eosin (HE), as well as Sudan.

After this time point, inspection of the cut open aorta under a binocular microscope showed atherosclerotic plaques that had the same extent in both, ApoE-/- mice immunized against IL-1β antiserum, or control ApoE-/- mice.

### EXAMPLE 16

### Passive immunization against IL-1β remained without effect on atherosclerosis

C57BL/6 mice were actively immunized against IL-1β with 3 subcutaneous injections of IL-1β-PPD conjugate in alum. After 56 days their serum was collected and generation of anti-IL-1β autoantibody titers were confirmed by ELISA. Two hundred µl of such serum was passively transferred to 6 weeks old ApoE knock out mice. These passive serum transfers were repeated every week. Control ApoE-/- mice received 200 µl serum transfers from naive C57BL/6 mice. Starting with these passive serum transfers, the ApoE-/- mice were fed an atherogenic diet with food pellets containing 16% fat, 1.16% cholesterol and 0.5 % cholic acid, in order to accelerate the formation of atherosclerosis. Control ApoE-/- mice were passively transferred 200 ml of serum from naive C57BL/6 mice in weekly intervals. Mice were euthanized after 6 weeks for macroscopic and histological analysis of the aorta. Histological analysis included haematoxylin and eosin staining of cross sections, as well as Sudan stains.

After these 6 weeks, inspection of the cut open aorta under a binocular microscope showed atherosclerotic plaques that had the same extent in both, ApoE-/- mice receiving anti-IL-1β antiserum, or serum from naive mice.

### EXAMPLE 17

### ADCK-Antibody dependent complement mediated killing

C57BL/6 mice were actively immunized against IL-1a with 3 subcutaneous injections of IL-1α-PPD conjugate in alum. After 56 days their serum was collected and generation of anti-IL-1α autoantibody titers were confirmed by ELISA. Sera were heat inactivated. 50 µl of an EL-4 cell suspensions were plated into 96 well plates. To each of these wells 15 µl of 1:2 serial dilutions of the heat inactivated serum was added. Plates were then incubated for 20 minutes at 37°C. Then 25ml of murine serum were added to each well. After another 5h incubation at 37°C wells are photographed and then the cells counted in a counting chamber using trypan blue to distinguish dead from alive cells.

The polyclonal mouse-anti-mouseIL-1α antiserum mediated complement dependent killing of EL-4 tumor cells in a concentration dependent fashion. See FIG. 2.

## Claims

1. IL-1α for use in a method of treatment of atherosclerotic plaques by reducing said plaques by at least 10% in a mammal by inducing IL-1α autoantibodies.

2. IL-1α for use according to claim 1, wherein the IL-1α is linked to a virus-like particle.

3. IL-1α for use according to claim 1 or claim 2 wherein the mammal is a human.

4. IL-1α for use according to claim 1 or claim 2 wherein the IL-1α is recombinant IL-1α.

5. IL-1α for use according to claim 4 wherein the recombinant IL-1α is murine or human.

6. IL-1α for use according to claim 1 wherein the IL-1α is linked to a carrier.

7. IL-1α for use according to claim 6 wherein the carrier is purified protein derivative of tuberculin (PPD).

8. IL-1α for use according to claim 1 or claim 2 wherein the IL-1α is used in the presence of an adjuvant.

9. IL-1α for use according to claim 8 wherein the adjuvant is aluminum hydroxide.

## Patentansprüche

1. IL-1α zur Verwendung in einem Verfahren zur Behandlung von atherosklerotischen Plaques durch Reduzieren der Plaques um mindestens 10% in einem Säugetier durch Induzieren von IL-1α Autoantikörpern.

2. IL-1α zur Verwendung gemäß Anspruch 1, wobei das IL-1α mit einem virusähnlichen Partikel verbunden ist.

3. IL-1α zur Verwendung gemäß Anspruch 1 oder 2, wobei das Säugetier ein Mensch ist.

4. IL-1α zur Verwendung gemäß Anspruch 1 oder 2, wobei das IL-1α rekombinantes IL-1α ist.

5. IL-1α zur Verwendung gemäß Anspruch 4, wobei das rekombinante IL-1α murin oder human ist.

6. IL-1α zur Verwendung gemäß Anspruch 1, wobei das IL-1α mit einem Träger verbunden ist.

7. IL-1α zur Verwendung gemäß Anspruch 6, wobei der Träger gereinigtes Proteinderivat von Tuberkulin (PPD) ist.

8. IL-1α zur Verwendung gemäß Anspruch 1 oder 2, wobei das IL-1α in der Gegenwart eines Adjuvans verwendet wird.

9. IL-1α zur Verwendung gemäß Anspruch 8, wobei das Adjuvans Aluminiumhydroxid ist.

## Revendications

1. IL-1α destinée à être utilisée dans un procédé de traitement des plaques athéroscléreuses par réduction desdites plaques d'au moins 10 % chez un mammifère par induction d'autoanticorps anti-IL-1α.

2. IL-1α destinée à être utilisée selon la revendication 1 où l'IL-1α est liée à une particule analogue à un virus.

3. IL-1α destinée à être utilisée selon la revendication 1 ou la revendication 2 où le mammifère est un humain.

4. IL-1α destinée à être utilisée selon la revendication 1 ou la revendication 2 où l'IL-1α est une IL-1α recombinante.

5. IL-1α destinée à être utilisée selon la revendication 4 où l'IL-1α recombinante est murine ou humaine.

6. IL-1α destinée à être utilisée selon la revendication 1 où l'IL-1α est liée à un vecteur.

7. IL-1α destinée à être utilisée selon la revendication 6 où le vecteur est un dérivé protéique purifié (DPP) de tuberculine.

8. IL-1α destinée à être utilisée selon la revendication 1 ou la revendication 2 où l'IL-1α est utilisée en présence d'un adjuvant.

9. IL-1α destinée à être utilisée selon la revendication 8 où l'adjuvant est l'hydroxyde d'aluminium.
